Europäisches Patentamt

European Patent Office

Office européen des brevets

(19)

(11) Veröffentlichungsnummer: **0 271 722**
**A1**

## (12) EUROPÄISCHE PATENTANMELDUNG

(21) Anmeldenummer: **87116806.8**

(22) Anmeldetag: **13.11.87**

(51) Int. Cl.⁴: **A61N 1/08** , A61N 1/36

(30) Priorität: **26.11.86 DE 3640350**

(43) Veröffentlichungstag der Anmeldung:
**22.06.88 Patentblatt 88/25**

(84) Benannte Vertragsstaaten:
**AT BE CH DE FR LI NL**

(71) Anmelder: **Siemens Aktiengesellschaft Berlin
und München
Wittelsbacherplatz 2
D-8000 München 2(DE)**

(72) Erfinder: **Sieber, Gotthold, Dipl.-Ing.
Hedenusstrasse 29
D-8520 Erlangen(DE)**

(54) **Reizstromgeraet.**

(57) Reizstromgerät, insbesondere für Reizstromtherapie an einem Patienten, mit einem Reizsignalerzeuger (2), der an Reizelektrodenanschlußbuchsen
(10, 11) eine vorgebbare Spannung anlegt, sowie mit
einer Stromabtasteinrichtung (14 bis 16) für Istwerte
des auf Grund der Spannung erzeugten Stromes.
Der Stromabtasteinrichtung ist eine Nullstromerfassungseinrichtung (4, 18, 19) nachgeschaltet, die ein
Ausgangssignal (S) wenigstens zum Zwecke einer
Abschaltung des inneren Stromkreises (12, 13) erzeugt, wenn für eine vorgebbare Zeitdauer (z.B. 2
Minuten) der Strom trotz eingeschalteter Spannung
Null ist.

## Reizstromgerät

Die Erfindung bezieht sich auf ein Reizstromgerät gemäß Oberbegriff des Patentanspruchs 1.

Bei Reizstromgeräten dieser Art muß sichergestellt werden, daß am Ende einer Behandlung die abgenommenen Reizstromelektroden nicht unnötig lange unter Spannung stehen, wenn die Bedienungsperson vergißt, das Gerät abzuschalten oder die Intensität auf Null zu stellen. Bei relativ hoher Spannung könnte es nämlich zu - schmerzhaften Sensationen kommen, wenn der Patient oder die Bedienungsperson mit den abgenommenen Elektroden in Berührung kommt. Dasselbe gilt auch für handgriffbetriebene Reizstromelektroden. Im Augenblick des Einschaltens am Handgriff liegt dann die volle hohe Spannung an den Elektroden. Das Problem ist insbesondere auch bei Konstantspannungsbetrieb relevant.

Aufgabe vorliegender Erfindung ist es, bei einem Reizstromgerät der eingangs genannten Art Mittel vorzusehen, die die Spannung abschalten, wenn zu vermuten ist, daß die Behandlung beendet ist und nicht, wie es bei Normalbehandlung häufiger vorkommt, die Elektroden lediglich zeitweilig abgehoben werden.

Die Aufgabe wird erfindungsgemäß durch die kennzeichnenden Merkmale des Patentanspruchs 1 gelöst.

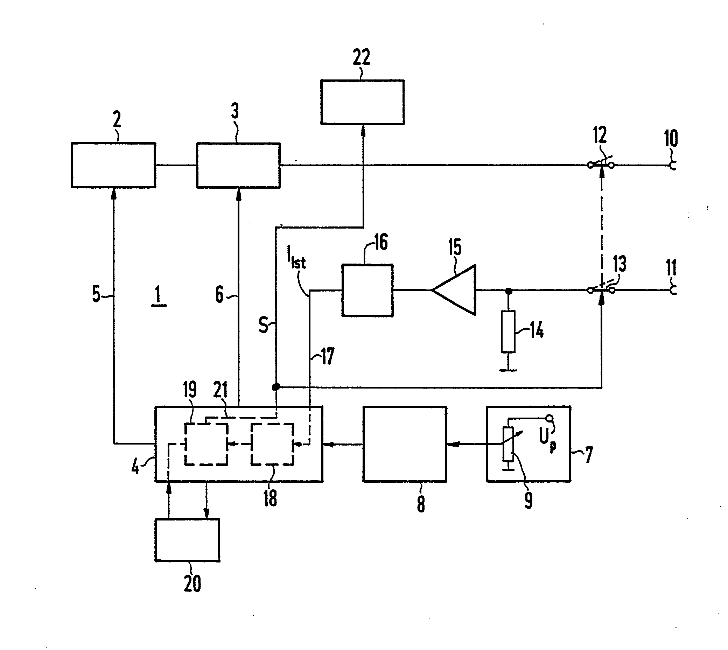

Weitere Vorteile und Einzelheiten der Erfindung ergeben sich aus der nachfolgenden Beschreibung eines Ausführungsbeispiels anhand der Zeichnung und in Verbindung mit den Unteransprüchen.

Die Figur zeigt das erfindungsgemäße Reizstromgerät im Prinzipschaltbild.

Das Reizstromgerät 1 umfaßt unter anderem einen Reizsignalerzeuger 2 mit Intensitätseinstelleinrichtung 3, die von einem Mikroprozessor 4 über Leitungen 5, 6 gesteuert werden. Der Intensitätseinstelleinrichtung 3 ist dabei über den Mikroprozessor 4 ein Intensitätseinstellglied 7 mit vorgeschaltetem Analog-Digital-Wandler 8 zugeordnet. Das Intensitätseinstellglied 7 umfaßt als Einstellmittel ein Drehpotentiometer 9 mit Drehknopf (nicht dargestellt). Die Betriebsspannung des Drehpotentiometers ist mit $U_P$ bezeichnet.

Die Buchsen 10, 11 sind die Geräteanschlußbuchsen für die (nicht dargestellten) Reizstromelektroden. Die (Relais) Schaltkontakte 12, 13 unterbrechen bei Betätigung (gestrichelte Stellung) den inneren Stromkreis des Reizstromgerätes 1. Damit kann bei Unterbrechung kein Reizstrom zum über die Elektroden angeschlossenen Patienten gelangen.

Zur Messung der Istwerte des Stromes beinhaltet das Reizstromgerät 1 ferner einen Meßwiderstand 14, dem über einen Verstärker 15 ein Analog-Digital-Wandler 16 nachgeschaltet ist. Der Analog-Digital-Wandler 16 tastet, gesteuert vom Mikroprozessor 4, den am Widerstand 14 anfallenden stromproportionalen Spannungsabfall ca. 20 mal pro Sekunde ab (Istwerte des Stromes).

Jeder über die Leitung 17 erfaßte Istwert $I_{Ist}$ wird vom Mikroprozessor 4 einer Nullstromdetektoreinheit 18 des Mikroprozessors 4 zugeleitet. Ist der Strom bei eingeschalteter Spannung Null, so startet eine Zeitzähleinheit 19 des Mikroprozessors 4. Die Zeitzähleinheit 19 bleibt aktiv, solange der Strom Null ist. Ist nach Ablauf einer vorgegebenen Zeitdauer, z.B. 2 Minuten, (eingespeichert in EPROM 20), der Strom immer noch Null, so erzeugt die Zeitzähleinheit 19 am Ausgang 21 ein Signal S. Das Signal S betätigt die Schaltkontakte 12, 13 im Sinne einer Abschaltung des inneren Stromkreises. Darüber hinaus wird ein optischer und akustischer Alarmgeber 22 durch das Signal aktiviert.

### Ansprüche

1. Reizstromgerät, insbesondere für Reizstromtherapie an einem Patienten, mit einem Reizsignalerzeuger, der an Reizelektrodenanschlußbuchsen eine vorgebbare Spannung anlegt, sowie mit einer Stromabtasteinrichtung für Istwerte des auf Grund der Spannung erzeugten Stromes, **dadurch gekennzeichnet,** daß der Stromabtasteinrichtung (14 bis 16) eine Nullstromerfassungseinrichtung (4, 18, 19) nachgeschaltet ist, die ein Ausgangssignal (S) wenigstens zum Zwecke einer Abschaltung des inneren Stromkreises (12, 13) erzeugt, wenn für eine vorgebbare Zeitdauer (z.B. 2 Minuten) der Strom trotz eingeschalteter Spannung Null ist.

2. Reizstromgerät nach Anspruch 1, **dadurch gekennzeichnet,** daß das Ausgangssignal (S) auch einen optischen und/oder akustischen Alarmgeber (22) aktiviert.

3. Reizstromgerät nach Anspruch 1 oder 2, **dadurch gekennzeichnet,** daß die Nullstromerfassungseinrichtung (4, 18, 19) eine Nullstromdetektoreinheit (18) sowie eine Zeitzähleinheit (19) als Bestandteil eines Mikroprozessors (4) umfaßt.

86 P 3431

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-4 177 819 (KOFSKY) <br> * Spalte 1, Zeilen 56-61; Spalte 4, Zeilen 15-54; Spalte 6, Zeilen 12-17; Figuren 1,5,8 * <br> ----- | 1-3 | A 61 N 1/08 <br> A 61 N 1/36 |

| RECHERCHIERTE SACHGEBIETE (Int. Cl.4) |
|---|
| A 61 N |

Der vorliegende Recherchenbericht wurde für alle Patentansprüche erstellt

| Recherchenort | Abschlußdatum der Recherche | Prüfer |
|---|---|---|
| DEN HAAG | 02-03-1988 | LEMERCIER D.L.L. |